(19) Europäisches Patentamt
European Patent Office
Office européen des brevets

(11) EP 0 667 151 B1

## (12) EUROPÄISCHE PATENTSCHRIFT

(45) Veröffentlichungstag und Bekanntmachung des Hinweises auf die Patenterteilung:
**22.04.1998 Patentblatt 1998/17**

(51) Int. Cl.⁶: **A61K 31/19**, A61K 9/20, A61K 9/00, A61K 47/12

(21) Anmeldenummer: **95100544.6**

(22) Anmeldetag: **16.01.1995**

(54) **Orale Arzeimittelzubereitung enthaltend Diclofenac-Natrium und eine organische Säure**

Oral drug formulation containing diclofenac sodium and an organic acid

Médicament à usage orale contenant le diclofenac sodium et un acide organique

(84) Benannte Vertragsstaaten:
**AT BE CH DE DK ES FR GB GR IE IT LI LU MC NL PT SE**
Benannte Erstreckungsstaaten:
**LT SI**

(30) Priorität: **08.02.1994 DE 4403943**

(43) Veröffentlichungstag der Anmeldung:
**16.08.1995 Patentblatt 1995/33**

(73) Patentinhaber: **HEXAL AG**
**D-83607 Holzkirchen (DE)**

(72) Erfinder: **Fischer, Wilfried, Dr.**
**D-83607 Holzkirchen (DE)**

(74) Vertreter:
**Boeters, Hans Dietrich, Dr. et al**
**Patentanwälte Boeters & Bauer,**
**Bereiteranger 15**
**81541 München (DE)**

(56) Entgegenhaltungen:
**EP-A- 0 209 975**        **EP-A- 0 553 777**
**WO-A-93/13760**        **DE-A- 3 909 520**
**DE-A- 3 915 150**

**Beschreibung**

Diclofenac ([2-(2,6-Dichloranilino)-phenyl]-essigsäure) ist in Form seines Natriumsalzes das am häufigsten verwendete nichtsteroidale Antirheumatikum. Die Substanz ist in Form von magensaftresistenten Tabletten oder Retardtabletten für die orale Anwendung in vielen pharmazeutischen Präparaten im Handel. Diclofenac zeichnet sich durch eine schnelle und zuverlässige Wirkung und relativ geringe Häufigkeit von Nebenwirkungen aus. Es bestehen jedoch Nachteile, da

1. Diclofenac bei den am häufigsten verwendeten Präparaten erst nach der Passage des Magens freigesetzt wird und daher zum Teil nur nach erheblicher Verzögerung absorbiert werden kann;

2. Diclofenac vor allem in retardierter Form bei der primären Leberpassage durch die Leber aus dem Blut herausgefiltert wird und, ohne klinisch wirksam zu werden, metabolisch eliminiert wird.

Insbesondere die magensaftresistenten Tabletten können große Probleme in der Zuverlässigkeit des Eintritts der Wirkung dadurch machen, daß die Aufenthaltsdauer von diesen überzogenen Tabletten nach Nüchterngabe im Bereich von 0 bis 4 Stunden liegt, mit den Mahlzeiten genommen jedoch im Bereich von 7 bis 16 Stunden, je nach Typ der Mahlzeit. Dieses ist eine prinzipbedingte Eigenschaft magensaftresistent überzogener einzeldosierter Arzneiformen. Sie hängt mit der Entleerungskinetik des Mageninhaltes zusammen. Formlinge, die größer als etwa 2 bis 4 mm sind, werden häufig im Magen festgehalten, wenn sie nicht zu kleineren Partikeln zerfallen. Der Schließmuskel des Magens, der Pylorus, schließt sich nämlich im allgemeinen, wenn Partikel ihn passieren, die einen größeren Durchmesser als eben diese 2 bis 4 mm haben. Diese Formen werden erst aus dem Magen herausgelassen, wenn die Nahrungsbestandteile ihn bereits verlassen haben.

Zur Behandlung der rheumatischen Erkrankungen ist es jedoch unerläßlich, reproduzierbare Blutspiegelverläufe mit sehr schnellem Wirkungseintritt zu erhalten. In dem Sinne ist es also besser, kleine Partikel unter 2 mm, die Diclofenac enthalten, zu applizieren, um eine rasche Magenentleerung zu erhalten. Ein entsprechendes Pelletpräparat - bestehend aus einer schnellfreisetzenden und einer retardierten Dosis - ist bereits im Handel (K. Brune, Fortschritt der Medizin, 109, Nr. 28, Seite 91 - 95 (1991)). Hierbei handelt es sich um magensaftresistent überzogene Pellets, die ihren Wirkstoff nach Verlassen des Magens schnell freisetzen, in Mischung mit magensaftresistent überzogenen Retardpellets, die nach Verlassen des Magens eine langsame Freisetzung ermöglichen. Auch diese galenische Form, obwohl sie eine reproduzierbarere Magenentleerungskinetik aufweist als Tabletten, ermöglicht es nicht, eine maximal schnelle Anflutung von Wirkstoff im Blut zu erhalten. Die Pellets müssen erst aus dem Magen entleert werden, was im Mittel etwa 1 Stunde dauern kann, bevor sie ihren Wirkstoff freigeben. Ein Rheumapatient mit Schmerzen möchte jedoch sehr schnell nach Einnahme seines Medikamentes eine Wirkung verspüren. Eine Verzögerungszeit von etwa 1 Stunde ist dabei oft nicht akzeptabel.

Die Art der Herstellung dieser magensaftresistenten Retardpellets ist sehr kompliziert, und der Patient muß eine relativ große Hartgelatinekapsel einnehmen, was speziell bei älteren Rheumapatienten, insbesondere mit Schluckbeschwerden, zu großen Problemen führen kann, so daß diese Medikation von vielen Patienten nicht appliziert werden kann.

Eine Verbesserung der Arzneiform, die allen therapeutischen Aspekte der Rheumabehandlung mit oralen Diclofenac-Arzneiformen Rechnung trägt, wäre eine nicht magensaftresistent überzogene, magenverträgliche Tablette, die geschluckt oder bei Patienten mit Schluckbeschwerden vor der Applikation in Wasser dispergiert und so getrunken werden kann. Nun beschreibt EP-A-0 365 480 eine schnellzerfallende Tablette, die Diclofenacsäure in einer Teilchengröße von 4 bis 100 μm enthält und nicht magensaftresistent überzogen ist. Diese Erfindung nutzt die Tatsache, daß Diclofenac als Säure in Wasser unlöslich ist und deshalb nicht den sehr schlechten, bitteren Geschmack des wasserlöslichen Diclofenac-Natrium aufweist. Damit ist diese Tablette vor der Applikation in Wasser dispergierbar, und eine Forderung der Diclofenactherapie, nämlich die leichte Applikation, ist damit erfüllt. Als Nachteil dieser Erfindung kann gewertet werden, daß Diclofenac als Säure eingesetzt wird, die als wasserunlösliche Substanz zur Erreichung einer ausreichend schnellen Absorption micronisiert werden muß. Dieses ist ein teurer Verfahrensschritt, der die Kosten der Herstellung in starkem Maße erhöht. Weiterhin ist die Säure hydrophob und damit wenig mit Wasser benetzbar, so daß die Tablette ein Tensid enthalten muß. In der genannten Patentanmeldung ist es beispielsweise Natriumlaurylsulfat, das in dem Verdacht steht, biologische Membranen, wie die Magenschleimhaut, zu schädigen.

Durch DE-A-3 909 520 (Beispiel 4) wird eine Brausetablette als Diclofenac-Na-Darreichungsform mit einem Gehalt an Zitronensäure und Natriumhydrogencarbonat beschrieben, die in Wasser zerfällt, wobei Diclofenac-Na, das von einem magensaftlöslichen Copolymerisat (Eudragit) umhüllt ist, im wässrigen Medium suspendiert wird. Diese bekannte Darreichungsform ist durch die Copolimerisat-Hülle zwar nicht mit dem unerwünschten Diclofenac-Na-Geschmack verhaftet, zeigt jedoch die für Retardformen (vergleiche Spalte 2 Zeile 44) angesprochenen Nachteile.

Ferner wird durch DE-A-3 909 520 (Beispiel 8) eine Brausetablette als Diclofenac-Darreichungsform mit einem Gehalt an Zitronensäure und Natriumhydrogencarbonat beschrieben, die in Wasser zerfällt, wobei diesmal das als Säure eingetzte Diclofenac einer Partikelgröße < 200 μm und mit einer Hülle aus Polyvinylpyrrolidon oder einer veretherten Zellulose im wässrigen Medium freigesetzt wird. Auch diese Darreichungsform ist nicht mit dem unerwünschtn Diclofenac-Na-Geschmack behaftet, allerdings wird wie bei der Lehre gemäß EP-A-0 365 480 aufwendig mikronisierter Wirkstoff eingesetzt, der in einem zusätzlichen Arbeitsschritt mit Polyvinylpyrrolidon überzogen werden muß.

DE-A-3 915 150 (Beispiel 1) beschreibt eine langwirkende Diclofenac-Natrium-Präparation, für die man in einem ersten Schritt ein Gemisch aus Diclofenac-Natrium und einer Säure, nämlich Zitronensäure, zu sphärischem Granulat verarbeitet, das man in einem zweiten Schritt mit einer die Wirkstofffreigabe verzögernden Masse sprühbeschichtet. Kommt wässriges Medium mit dem als Granulat vorliegenden Gemisch aus Diclofenac-Natrium und Zitronensäure in Berührung, so kommt es auf der Granulatoberfläche unter der freisetzungsverzögernden Schicht zur Bildung von Diclofenac-Säure. Demnach wird entsprechend obengenannter Lehren die Säure immer in Zusammenhang mit Retardformen verwandt, da aufgrund der geringen Löslichkeit der Säure die Resorptionsgeschwindigkeit ebenfalls niedrig ist.

Bei einer anderen Ausführungsform nach DE-A-3 915 150 (Beispiel 2) wird das vorstehend angeführte langwirkende Granulat aus Diclofenac-Natrium und Säure als Kern verwendet, auf den Diclofenac-Natrium zu sphärischem Granulat aufgebracht wird, das wiederum mit einer die Wirkstofffreigabe verzögernden Beschichtung versehen wird. Der kurzwirkende Diclofenac-Natrium-Anteil der fertigen Präparation enthält keine Säure.

Bei einer weiteren Ausführungsform nach DE-A-3 915 150 (Beispiel 9) wird eine langwirkende Diclofenac-Natrium-Präparation dadurch hergestellt, daß man in einem ersten Schritt ein Gemisch aus Diclofenac-Natrium und Fumarsäure zu Granulat verarbeitet, das man in einem zweiten Schritt wiederum mit einer die Wirkstofffreisetzung verzögernden Masse sprühbeschichtet. Eine analoge Ausführungsform findet sich in Beispiel 10.

EP-A-0 553 777 beschreibt eine Tablette für eine Reihe von Wirkstoffen wie auch u. a. Diclofenac-Natrium und mit einem Gehalt an diversen Zusätzen wie Sprengmitteln, Bindemitteln, Treibmitteln, Süßstoffen, Duftstoffen, Gleitmitteln, Farbstoffen und Säuren, beispielsweise Zitronensäure. Das Problem, dem unangenehmen Geschmack von Diclofenac-Natrium Rechnung zu tragen, und eine Kombination dieses Wirkstoffs mit einer organischen Säure werden jedoch nicht angesprochen.

Aus WO-A-93/13 760 ist eine pharmazeutische Zubereitung mit einem Gehalt an beispielsweise Diclofenac-Natrium und Zitronensäure bekannt, bei der der unangenehme Geschmack des Wirkstoffs durch Einbau in eine Matrix (Seite 2 Zeile 10) maskiert werden soll (Seite 5 Zeile 15).

Es besteht damit weiterhin Bedarf an einer gut verträglichen und schnell wirksamen, leicht applizierbaren Diclofenaczubereitung. Aufgabe der vorliegenden Erfindung ist es nun, eine preiswerte Arzneiform mit dem üblichen Diclofenac-Natrium zur Verfügung zu stellen, die einen maximal schnellen Wirkungseintritt ermöglicht, dabei sowohl unverändert applizierbar, als auch nach vorheriger Dispersion in Wasser leicht einzunehmen sein soll. Insbesondere soll dabei auf die Verwendung des üblichen Diclofenac-Natrium, das in großen Mengen zu günstigen Preisen erhältlich ist, Wert gelegt werden.

Die der Erfindung zugrundeliegende Aufgabe wird gemäß einer Ausführungsform durch eine Arzneimittelzubereitung zu oraler Applikation (ohne vorherige Auflösung in einem wäßrigen Medium) gelöst, die einen maximal schnellen Wirkungseintritt bietet und einen Gehalt an Diclofenac-Natrium sowie einer pharmakologisch akzeptablen organischen Säure, die nach Auflösen in einem wäßrigen Medium einen pH-Wert der anfallenden wäßrigen Zubereitung ≤ 4 ergibt, jedoch keinen magensaftresistenten Überzug und keinen wasserresistenten Überzug der Zubereitung oder der Wirkstoffpartikel aufweist.

Gemäß einer weiteren Ausführungsform wird die der Erfindung zugrundeliegende Aufgabe durch eine wäßrige Arzneimittelzubereitung zu oraler Applikation gelöst, die einen maximal schnellen Wirkungseintritt bietet und erhältlich ist durch Auflösen einer Zubereitung mit einem Gehalt an Diclofenac-Natrium sowie einer pharmakologisch akzeptablen organischen Säure, ohne einen magensaftresistenten Überzug und ohne einen wasserrestistenten Überzug der Zubereitung oder der Wirkstoffpartikel aufzuweisen, wobei die organische Säure nach Auflösen einen pH-Wert der wäßrigen Arzneimittelzubereitung ≤ 4 ergibt.

Überraschenderweise ergibt Diclofenac-Natrium in Kombination mit einer organischen Säure nach Dispersion in Wasser eine geschmacklose und damit für den Patienten angenehme Wirkstoffsuspension. Weiterhin überraschend ist dabei, daß die sehr hydrophobe Diclofenac-Säure, die bei dieser Dispersion anfällt, sich sehr gut benetzen läßt und in sehr feiner Teilchenform anfällt (etwa 1 bis 5 μm), so daß eine extrem schnelle Resorption aus dem Magen-Dünndarm-Bereich erreicht wird. Da im Magen nur sehr geringe Wirkstoffkonzentrationen erreicht werden, ist eine gute Magenverträglichkeit ohne speziellen magensaftresistenten Überzug gewährleistet.

Essentiell ist also das Vorhandensein einer organischen Säure, die nicht flüchtig sein sollte. Man kann dazu als organische Säure beispielsweise Zitronensäure, Bernsteinsäure oder Adipinsäure wählen.

Die erfindungsgemäße Arzneimittelzubereitung kann ein inertes Füllmittel und/oder Trägermaterial enthalten, beispielsweise Laktose oder Stärke. Ferner kann die erfindungsgemäße Arzneimittelzubereitung ein inertes Bindemittel enthalten, beispielsweise Polyvinylpyrrolidon (PVP).

Die erfindungsgemäße Arzneimittelzubereitung kann als Granulat vorliegen, beispielsweise als schnell zerfallendes Granulat, das beispielsweise aus inerten Trägermaterialien, wie Laktose oder Stärke, einem Bindemittel, wie Polyvinylpyrrolidon, Diclofenac-Natrium und einer organischen Säure besteht. Das Granulat kann in Kapseln oder in Säckchen (Saches) abgefüllt werden, die man vor Applikation in Wasser dispergiert. Bei Abfüllung in Kapseln können die Kapseln auch ohne vorherige Dispersion des Inhalts direkt appliziert werden.

Ferner kann die erfindungsgemäße Arzneimittelzubereitung als Tablette vorliegen Derartige Tabletten können ein Sprengmittel, beispielsweise modifizierte Stärke, quervernetztes Polyvinylpyrrolidon (PVP), Zellulosederivat oder gasbildenden Stoff, insbesondere Natriumhydrogencarbonat enthalten. Dadurch kann die Zerfallszeit der Tabletten reduziert werden. Die erfindungsgemäßen Tabletten können beispielsweise auch als Brausetabletten vorliegen. Derartige Tabletten können vom Patienten direkt eingenommen oder vor Einnahme in Wasser dispergiert werden.

Die Tabletten können dabei beispielsweise durch herkömmliche Feuchtgranulation in einem Zwangswäscher oder durch Wirbelschichtgranulation und anschließendes Verpressen oder durch Direkttablettierung unter Umgehung der Granulation hergestellt werden.

Gemäß einer speziellen Ausführungsform wird die erfindungsgemäße Arzneimittelzubereitung als Mehrschichttablette, beispielsweise als Zweischichttablette vorgesehen, wobei eine Schicht für Diclofenac-Natrium und die andere Schicht für die organische Säure bestimmt ist.

Ein Netzmittel kann vorhanden sein, ist aber nicht Bedingung für die Funktionsfähigkeit der erfindungsgemäßen Arzneimittelzubereitung.

Die folgenden Beispiele sollen die Erfindung verdeutlichen:

**Beispiel 1**: Dispergierbare Diclofenac-Natrium-Tabletten

| | |
|---|---|
| Diclofenac-Natrium | 50 mg |
| Lactose | 132 mg |
| Maisstärke | 35 mg |
| PVP, quervernetzt | 8,5 mg |
| Bernsteinsäure | 50 mg |
| Magnesiumstearat | 3,5 mg |
| Kolloidales Siliciumdioxid | 3,5 mg |
| mikrokristalline Cellulose | 70 mg |
| | 362,5 mg |

Obengenannte Bestandteile werden homogen gemischt und auf einer Rundlauftablettenmaschine zu Tabletten mit einem Gesamtgewicht von 362,5 mg entsprechend 50 mg Natriumdiclofenac verpreßt.

**Beispiel 2**: Dispergierbare Diclofenac-Natrium-Tabletten

| | |
|---|---|
| Diclofenac-Natrium | 50 mg |
| mikrokristalline Cellulose | 30 mg |
| Lactose | 60 mg |
| PVP | 10 mg |
| Zitronensäure | 50 mg |
| Kolloidales Siliciumdioxid | 5 mg |
| Aroma | 7 mg |
| Magnesiumstearat | 5 mg |
| | 217 mg |

Die Bestandteile Natriumdiclofenac, Lactose, Maisstärke und Bernsteinsäure werden mit einer wässerigen Lösung des PVP in der Wirbelschicht granuliert, getrocknet und nach Ausmischem mit Magnesiumstearat zu Tabletten mit einem Gesamtgewicht von 217 mg entsprechend 50 mg Diclofenac verpresst. Diese Tabletten können vorzugsweise eingenommen, aber auch vor der Einnahme in Wasser dispergiert werden.

**Beispiel 3**: Diclofenac-Natrium-Trinkgranulat

| | |
|---|---|
| Diclofenac Natrium | 50 mg |
| Saccharose | 150 mg |
| Zitronensäure | 60 mg |
| lösliche Stärke | 5 mg |
| Aerosil | 1,5 mg |

Diclofenac-Natrium, Saccharose und Zitronensäure werden gemischt und in einem Zwangsmischer mit einer Dispersion der löslichen Stärke in Wasser granuliert. Das Granulat wird bei 40 °C auf Horden getrocknet, mit Aerosil ausgemischt und in Saches abgefüllt und verschlossen. Dieses Granulat wird vor der Einnahme in einer geringen Menge kaltem Wasser, z. B. 100 ml, dispergiert und eingenommen.

**Beispiel 4**: Diclofenac-Natrium-Zweischichttablette

| Schicht 1 | | Schicht 2 | |
|---|---|---|---|
| Diclofenac-Natrium | 25 mg | Citronensäure | 25 mg |
| Lactose | 15 mg | Lactose | 15 mg |
| Calciumphosphat | 20 mg | Calciumphosphat | 20 mg |
| mikrokristalline Cellulose | 24,5 mg | mikrokristalline Cellulose | 24,5 mg |
| Maisstärke | 10 mg | Maisstärke | 10 mg |
| modifizierte Maisstärke | 4 mg | modifizierte Maisstärke | 4 mg |
| Magnesiumstearat | 1 mg | Magnesiumstearat | 1 mg |
| Kolloidales Siliciumdioxid | 0,5 mg | Kolloidales Siliciumdioxid | 0,5 mg |

Die Bestandteile der Schichten 1 und 2 werden separat homogen gemischt und auf einer Rundlauftablettenpresse zu Zweischichttabletten zu 200 mg verpreßt.

**Anwendungsbeispiel 1:**

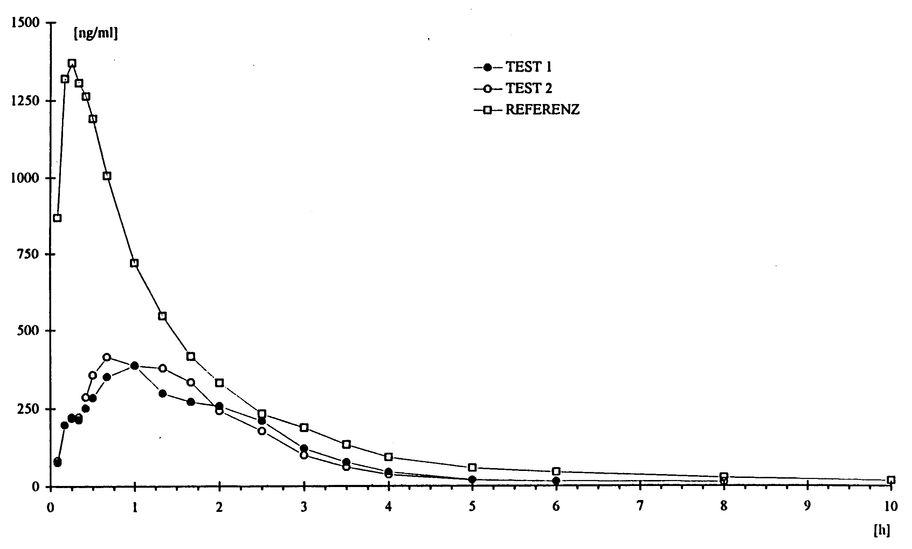

Jeweils 12 Probanden wurde eine erfindungsgemäße Diclofenac-Natrium-Tablette gemäß Beispiel 1 (Test 1), eine Diclofenac-Tablette des Handels mit mikronisierter Diclofenac-Säure und Tensid (Test 2) sowie eine intramuskuläre Injektion einer Diclofenac-Natrium-Lösung (Referenz) verabreicht. **Figur 1** sind die Mittelwertsverläufe des Blutplasmaspiegels zu entnehmen.

**Patentansprüche**

1. Arzneimittelzubereitung zu oraler Applikation (ohne vorherige Auflösung in einem wäßrigen Medium) mit maximal schnellem Wirkungseintritt und mit einem Gehalt an Diclofenac-Natrium sowie einer pharmakologisch akzeptablen organischen Säure, die nach Auflösen in einem wäßrigen Medium einen pH-Wert der anfallenden wäßrigen Zubereitung ≤ 4 ergibt, jedoch ohne magensaftresistenten Überzug und ohne wasserresistenten Überzug der Zubereitung oder der Wirkstoffpartikel.

2. Wäßrige Arzneimittelzubereitung zu oraler Applikation mit maximal schnellem Wirkungseintritt, erhältlich durch Auflösen einer Zubereitung mit einem Gehalt an Diclofenac-Natrium sowie einer pharmakologisch akzeptablen organischen Säure, jedoch ohne magensaftresistenten Überzug und ohne wasserresistenten Überzug der Zubereitung oder der Wirkstoffpartikel, wobei die organische Säure nach Auflösen einen pH-Wert der wäßrigen Arzneimittel-Zubereitung ≤ 4 ergibt.

3. Arzneimittelzubereitung nach Anspruch 1, **gekennzeichnet** durch einen Gehalt an einem inserten Füllmittel und/oder Trägermaterial, beispielsweise Lactose oder Stärke.

4. Arzneimittelzubereitung nach einem der Ansprüche 1 oder 3, **gekennzeichnet** durch einen Gehalt an einem inerten Bindemittel, beispielsweise Polyvinylpyrrolidon (PVP).

5. Arzneimittelzubereitung nach einem der Ansprüche 1 oder 3 bis 4, dadurch **gekennzeichnet**, daß sie als Granulat vorliegt.

6. Arzneimittelzubereitung nach einem der Ansprüche 1 oder 3 bis 5, dadurch **gekennzeichnet**, daß sie als Granulat abgefüllt in Kapseln oder Säckchen (Saches) vorliegt.

7. Arzneimittelzubereitung nach einem der Ansprüche 1 oder 3 bis 6, dadurch **gekennzeichnet**, daß sie als Tablette vorliegt.

8. Arzneimittelzubereitung nach Anspruch 7, **gekennzeichnet** durch einen Gehalt an einem nicht-gasbildenden Sprengmittel, beispielsweise modifizierte Stärke, quervernetztes Polyvinylpyrrolidon (PVP) oder Cellulosederivat.

9. Arzneimittelzubereitung nach einem der Ansprüche 1 oder 3 bis 8, **gekennzeichnet** durch einen Gehalt an einem gasbildenden Stoff, insbesondere Natriumhydrogencarbonat.

10. Arzneimittelzubereitung nach Anspruch 9, dadurch **gekennzeichnet**, daß sie als Brausetablette vorliegt.

11. Arzneimittelzubereitung nach einem der Ansprüche 7 bis 10, dadurch **gekennzeichnet**, daß sie als Mehrschichttablette, beispielsweise als Zweischichttablette, vorliegt, wobei eine Schicht für Diclofenac-Natrium und die andere Schicht für die organische Säure vorgesehen ist.

12. Arzneimittelzubereitung nach einem der vorhergehenden Ansprüche, **gekennzeichnet** durch Zitronensäure, Bernsteinsäure oder Adipinsäure als organische Säure.

## Claims

1. Medicinal preparation for oral application (without previous dissolution in an aqueous medium) taking effect with the maximum speed and with a diclophenac-sodium content as well as with a pharmacologically acceptable organic acid which after dissolution in an aqueous medium results in a pH value of ≤ 4 for the aqueous preparation occurring but without any coating resistant to the gastric juices and without any water-resistant coating for the preparation or for the particles of active substance.

2. Aqueous medicinal preparation for oral application, taking effect with the maximum speed, obtainable by dissolution of a preparation with a diciclophenac-sodium content and with a pharmacologically acceptable organic acid, but without any coating resistant to the gastric juices and without any water-resistant coating for the preparation or for the particles of active substance, the organic acid resulting, after dissolution, in a pH value of ≤ 4 for the aqueous medicinal preparation.

3. Medicinal preparation according to Claim 1, characterized by a proportion of an inert filling agent and/or carrier material such as lactose or starch.

4. Medicinal preparation according to either of Claims 1, 3, characterized by a proportion of an inert binding agent, such as polyvinyl pyrrolidon (PVP)..

5. Medicinal preparation according to any one of Claims 1,3,4, characterized by the fact that it is present in granulate form.

6. Medicinal preparation according to any one of Claims 1,3-5, characterized by the fact that it is present in the form of granulate filled into capsule or sachets.

7. Medicinal preparation according to any one of Claims 1,2-6, characterized by the fact that it is present in the form of a tablet.

8. Medicinal preparation according to Claim 7, characterized by a proportion of a non-gasifying explosive agent, such as modified starch, cross-linked polyvinyl pyrrolidon (PVP) or cellulose derivative.

9. Medicinal preparation according to any one of Claims 1, 3-8, characterized by a proportion of gas-forming substance, particularly sodium hydrogen carbonate.

10. Medicinal preparation according to Claim 9, characterized by the fact that it is present in the form of an effervescent tablet.

11. Medicinal preparation according to any one of Claims 7-10, characterized by the fact that it is present in the form of a multi-layered tablet, one layer being provided for diclopenac sodium and the other for the organic acid.

12. Medicinal preparation according to any one of the foregoing Claims, characterized by the fact that the organic acid consists of succinic acid or adipic acid.

**Revendications**

1. Préparation médicamenteuse à usage oral (sans dissolution préalable dans un milieu aqueux) et à effet très rapide, qui contient du sel de sodium de diclofénac ainsi qu'un acide organique admissible du point de vue pharmacologique, et qui donne, après dissolution dans un milieu aqueux, une préparation aqueuse dont le pH est inférieur ou égal à 4, mais qui ne comporte aucun enrobage résistant au suc gastrique ni aucun enrobage résistant à l'eau, sur la préparation ou sur les particules de substance active.

2. Préparation médicamenteuse aqueuse à usage oral et à effet très rapide, qu'on peut obtenir par dissolution d'une préparation contenant du sel de sodium de diclofénac et un acide organique admissible du point de vue pharmacologique, mais ne comportant aucun enrobage résistant au suc gastrique ni aucun enrobage résistant à l'eau, sur la préparation ou sur les particules de substance active, l'acide organique conférant à la préparation médicamenteuse aqueuse, après dissolution, un pH inférieur ou égal à 4.

3. Préparation médicamenteuse conforme à la revendication 1, caractérisée en ce qu'elle contient une charge inerte et/ou un véhicule, par exemple du lactose ou de l'amidon.

4. Préparation médicamenteuse conforme à l'une des revendications 1 et 3, caractérisée en ce qu'elle contient un liant inerte, par exemple de la poly(vinyl-pyrrolidone) (PVP).

5. Préparation médicamenteuse conforme à l'une des revendications 1, 3 et 4, caractérisée en ce qu'elle se présente en granulés.

6. Préparation médicamenteuse conforme à l'une des revendications 1 et 3 à 5, caractérisée en ce qu'elle se présente en granulés mis dans des capsules ou dans des sachets.

7. Préparation médicamenteuse conforme à l'une des revendications 1 et 3 à 6, caractérisée en ce qu'elle se présente en comprimés.

8. Préparation médicamenteuse conforme à la revendication 7, caractérisée en ce qu'elle contient un agent délitant ne produisant pas de gaz, par exemple de l'amidon modifié, de la poly(vinyl-pyrrolidone) (PVP) réticulée ou un dérivé de cellulose.

9. Préparation médicamenteuse conforme à l'une des revendications 1 et 3 à 8, caractérisée en ce qu'elle contient une substance produisant un gaz, en particulier de l'hydrogénocarbonate de sodium.

10. Préparation médicamenteuse conforme à la revendication 9, caractérisée en ce qu'elle se présente en comprimés effervescents.

11. Préparation médicamenteuse conforme à l'une des revendications 7 à 10, caractérisée en ce qu'elle se présente en comprimés à plusieurs couches, par exemple en comprimés à deux couches où l'une des couches contient le sel de sodium de diclofénac et l'autre couche contient l'acide organique.

12. Préparation médicamenteuse conforme à l'une des revendications précédentes, caractérisée en ce que ledit acide organique est de l'acide citrique, de l'acide succinique ou de l'acide adipique.